# FASCICULE DE BREVET EUROPEEN

(11) **EP 1 245 246 B1**
(45) Date de publication et mention de la délivrance du brevet: **26.01.2005**
(21) Numéro de dépôt: 02290757.0
(22) Date de dépôt: 27.03.2002
(51) Int. Cl.: A61M 35/00, A45D 40/26, A47K 7/02, A41D 19/00, A61B 19/04

(54) **Dispositif de traitement comportant une enveloppe définissant une cavité dans laquelle peut être engagée une partie de corps**
Behandlungsvorrichtung in Form einer Verpackungshülle zum Einbringen eines Teils des menschlichen Körpers
Treatment device comprising an envelope defining a cavity within which a part of the human body can be inserted

(30) Priorité: 28.03.2001 FR 0104169
(43) Date de publication de la demande: 02.10.2002
(73) Titulaire: L'OREAL, 75008 Paris (FR)
(72) Inventeur: Gueret, Jean-Louis, 75016 Paris (FR)
(74) Mandataire: Tanty, François

(56) Documents cités:
- WO-A-01/10567
- US-A- 3 384 083
- US-A- 4 122 554
- US-A- 5 679 399
- US-A- 6 117 119

## Description

La présente invention concerne un dispositif de traitement d'une région du corps humain, par exemple les mains, le visage ou les cheveux.

Il existe un besoin pour bénéficier d'un dispositif de traitement qui soit d'une utilisation facile, agréable, et dont la fabrication soit relativement simple.

Les documents WO 01/10567 et US-A-6 117 119 divulguent des dispositifs de traitment selon le préambule de la revendication 1.

Le dispositif de traitement selon l'invention se caractérise par le fait qu'il comporte une matrice adhésive située entre deux couches dont l'une au moins est perméable à un solvant, ces deux couches étant liées directement ou indirectement de manière permanente à la matrice adhésive, cette dernière comprenant au moins un actif soluble dans ledit solvant, l'actif une fois dissout étant apte à être libéré au moins d'un côté de l'enveloppe.

Deux éléments sont liés de manière permanente lorsqu'ils ne peuvent pas être séparés aisément simplement par une force manuelle. Par exemple, un film détachable en contact avec un adhésif sur un bandage ne serait pas lié de manière permanente au reste du bandage.

Grâce à l'invention, le traitement peut s'effectuer de manière aisée après avoir mis toute ou partie de l'enveloppe au contact du solvant, qui peut être de l'eau par exemple.

Le traitement peut avoir lieu à l'intérieur de l'enveloppe ou à l'extérieur de l'enveloppe, ou les deux.

Lorsque le traitement a lieu à l'intérieur de l'enveloppe, l'actif est libéré au contact de la partie du corps disposée dans l'enveloppe, par exemple une main. L'enveloppe peut faciliter l'action du ou des actifs libérés au contact de la région traitée, renforcer l'efficacité du traitement et ajouter, le cas échéant, un effet thermique susceptible d'améliorer la pénétration du ou des actifs dans la peau.

Lorsque le traitement a lieu à l'extérieur de l'enveloppe, cette dernière permet d'utiliser le dispositif de traitement à la manière d'un gant de toilette par exemple et de combiner une action de massage avec une action d'application d'un ou plusieurs actifs et/ou avec une action de nettoyage de la peau.

L'enveloppe peut présenter la forme d'un gant, notamment d'une moufle, afin de permettre le traitement d'une main et/ou d'utiliser l'enveloppe à la manière d'un gant de toilette pour traiter une autre partie du corps.

L'enveloppe peut encore présenter la forme d'un bonnet destiné à coiffer la tête de l'utilisateur pour traiter les cheveux ou le cuir chevelu.

L'enveloppe peut en variante présenter la forme d'un doigt de gant afin de permettre le traitement d'un doigt.

On peut aussi réaliser l'enveloppe avec la forme d'une cagoule pour traiter les cheveux et/ou le visage, d'un chausson ou encore d'une poche à disposer autour d'une oreille, par exemple.

L'enveloppe peut être élastiquée, notamment autour de l'ouverture par laquelle l'intérieur de l'enveloppe communique avec l'extérieur, afin par exemple d'améliorer sa tenue sur la partie du corps engagée à l'intérieur ou amener l'enveloppe à former une poche.

L'enveloppe peut être réalisée à partir d'une feuille unique repliée sur elle-même ou conformée en poche ou à partir de deux ou plusieurs feuilles assemblées entre elles.

Lorsque deux feuilles sont assemblées, l'assemblage peut s'effectuer sur une partie seulement de la périphérie des feuilles, afin de ménager une ouverture pour l'introduction d'une partie du corps.

Les feuilles peuvent être assemblées par soudure, collage ou couture, notamment.

L'enveloppe peut comporter des fentes ou ouvertures disposées en quinconce à la manière d'un métal déployé, de façon à présenter une certaine extensibilité.

Dans une réalisation particulière, la matrice adhésive contient un ou plusieurs actifs solubles dans le solvant utilisé et/ou capables de gonfler au contact dudit solvant, ces actifs étant introduits avec une quantité suffisante dans la matrice pour que cette dernière perde de sa cohésion au contact du solvant et libère plus facilement le ou les actifs. En variante ou additionnellement, la matrice adhésive peut contenir une charge d'un ou plusieurs composés capables de gonfler au contact dudit solvant, cette charge étant introduite dans la matrice avec une quantité suffisante pour que celle-ci perde de sa cohésion au contact du solvant et libère plus facilement le ou les actifs. En variante ou additionnellement, la matrice adhésive peut encore contenir une charge d'un ou plusieurs composés sensiblement inertes, dans une quantité suffisante pour que la matrice perde de sa cohésion au contact du solvant et libère plus facilement le ou les actifs.

La matrice adhésive peut ainsi comporter un ou plusieurs composés absorbeurs d'humidité, par exemple entre 0,2 et 60 % en masse d'un composé absorbeur d'humidité, notamment entre 0,5 et 40 %, ce composé étant choisi par exemple parmi les polyacrylates, les silices, les fibres de coton, les amidons, les alginates, les carbonates de calcium ou de magnésium, la viscose, la cellulose, les lyophylisats, cette liste n'étant pas limitative. Outre des composés absorbeurs d'humidité ou en remplacement de ceux-ci, la matrice adhésive peut comporter une ou plusieurs substances capables d'abaisser son pouvoir adhésif et lui permettre d'éclater au contact du solvant afin de faciliter la libération du ou des actifs. Parmi de telles substances, on peut citer par exemple des substances sensiblement inertes telles que des microbilles ou une poudre d'un composé inerte, par exemple la poudre de polyamide connue sous la dénomination ORGASOL.

La matrice adhésive peut comporter un ou plusieurs actifs choisis dans la liste suivante : vitamine C, vitamine A, vitamine F, glycérine, laponite, tensioactifs, collagène, acide salicylique, acide tio, huiles essentielles aromatiques, colorants, caféine, anti-oxydants, anti-radicaux libres, hydratants, dépigmentants, liporégulateurs, anti-acnéiques, anti-séborrhéiques, anti-vieillissement, adoucissants, antirides, kératolitiques, anti-inflammatoires, rafraîchissants, cicatrisants, protecteurs vasculaires, anti-bactériens, anti-fongiques, anti-perspirants, déodorants, conditionneurs de la peau, insensibilisants, immunomodulateurs et nourrissants, cette liste n'étant pas limitative, bien entendu.

La matrice adhésive peut encore comporter des particules magnétisables ou magnétiques, destinées à améliorer la microcirculation. La structure composite peut notamment comporter au moins deux couches de particules magnétisables, capables de générer des champs magnétiques respectifs de polarités différentes.

Le solvant utilisé peut être autre que de l'eau, notamment une huile ou une lotion.

Le dispositif de traitement selon l'invention permet de conditionner aisément un ou plusieurs actifs en les incorporant dans une ou plusieurs matrices adhésives. La conservation peut s'effectuer à l'état anhydre dans de bonnes conditions puisque l'enveloppe peut être imprégnée de solvant seulement lors de l'utilisation. On peut ainsi ne pas utiliser de conservateurs ou en diminuer la teneur. Le conditionnement est également rendu plus simple.

La masse surfacique de la matrice peut être comprise entre 10 g/m² et 100 g/m² par exemple.

Dans une réalisation particulière, l'une des couches au contact de la matrice adhésive est constituée par un non-tissé ou comporte un non-tissé laminé avec un film préalable ou non, un filet, un voile, un tissé, une éponge, un feutre ou autre. La matrice adhésive peut être notamment située entre deux couches poreuses comprenant chacune un non-tissé par exemple.

D'une manière générale, les faces intérieure et extérieure de l'enveloppe peuvent présenter des rugosités différentes. Les couches entre lesquelles la matrice est située peuvent présenter des porosités ou épaisseurs différentes, afin de permettre deux types d'application différents, selon la face choisie par l'utilisateur, avec retournement éventuel sur elle-même de l'enveloppe. Les faces extérieure et intérieure de l'enveloppe peuvent présenter des couleurs différentes afin de faciliter leur repérage par l'utilisateur.

L'enveloppe peut présenter sur une face, par exemple sa face intérieure, un voile thermosoudable destiné à faciliter sa fabrication en permettant d'assembler la feuille sur elle-même ou avec une autre feuille présentant également un tel voile. Ce dernier peut être présent par exemple sur une face d'un non-tissé lié par son autre face à la matrice adhésive. Un tel voile n'est toutefois pas indispensable, la ou les feuilles constituant l'enveloppe pouvant être assemblées de multiples manières.

Dans une réalisation particulière, la structure composite comporte une couche imperméable ou partiellement occlusive (c'est-à-dire semi-perméable), afin par exemple de favoriser la diffusion d'un actif dans la peau en retardant l'évaporation du solvant. Cette couche imperméable ou partiellement occlusive peut être présente entre la matrice et l'extérieur de l'enveloppe lorsque le traitement doit avoir lieu à l'intérieur de l'enveloppe et contribuer alors à retenir dans l'enveloppe le solvant, ce qui est avantageux notamment lorsque le solvant est volatil ou n'est introduit dans l'enveloppe qu'en faible quantité.

La structure composite peut encore comporter au moins deux matrices adhésives juxtaposées ou superposées, de compositions identiques ou différentes. Il peut notamment être avantageux d'utiliser un assemblage de deux ou plusieurs matrices adhésives pour obtenir une combinaison d'actifs souhaitée plutôt que de chercher à incorporer tous les actifs dans la même matrice adhésive. En particulier, une matrice adhésive donnée peut être fabriquée en grande quantité avec un ou plusieurs actifs choisis puis assemblée avec une ou plusieurs matrices adhésives différentes, contenant d'autres actifs, pour constituer une gamme de structures composites ayant des propriétés variées.

Ainsi, dans un exemple de réalisation, la structure composite comporte, dans l'ordre, la superposition de couches suivante : une première couche perméable au solvant, une première matrice adhésive, une deuxième matrice adhésive et une deuxième couche perméable au solvant. Dans un autre exemple de réalisation, la structure composite comporte, dans l'ordre, la superposition de couches suivante : une première couche perméable au solvant, une première matrice adhésive contenant au moins un actif, une couche intermédiaire imperméable, une deuxième matrice adhésive contenant au moins un actif, et une deuxième couche perméable, les première et deuxième matrices adhésives contenant des actifs différents. De telles structures composites sont avantageusement fabriquées en enduisant séparément chaque couche perméable d'une matrice adhésive, puis en assemblant les différentes couches perméables ainsi revêtues, soit directement, soit autour d'une couche intermédiaire, imperméable dans l'exemple ci-dessus, mais pouvant également être perméable au solvant.

Dans un autre exemple de réalisation, la structure composite comporte une première matrice adhésive comprenant deux régions juxtaposées contenant des actifs différents. La structure composite peut éventuellement comporter en outre une deuxième matrice adhésive comprenant également deux régions juxtaposées contenant des actifs différents, éventuellement d'autres actifs que ceux contenus dans la première matrice adhésive. On peut ainsi multiplier aisément les combinaisons d'actifs au sein d'une même structure composite, et adapter la répartition des actifs à la nature et au positionnement des zones à traiter.

La structure composite peut être agencée de manière à favoriser la diffusion du ou des actifs contenus dans la matrice adhésive vers une face de l'enveloppe. Pour créer une diffusion préférentielle en direction d'une face de l'enveloppe définie par une couche perméable au solvant, par exemple un non-tissé, il est possible notamment de déposer la matrice adhésive à l'état fluide sur ladite couche perméable de manière à lui permettre de diffuser au moins partiellement entre les fibres ou particules de ladite couche, sur une partie au moins de l'épaisseur de celle-ci.

L'invention a encore pour objet un procédé de fabrication d'un dispositif de traitement d'une partie du corps, comportant les étapes suivantes :
- fournir au moins une feuille présentant une structure composite comportant au moins une matrice située entre deux couches dont l'une au moins est perméable à un solvant, ces deux couches étant liées de manière permanente à la matrice adhésive, cette dernière comprenant au moins un actif soluble dans le solvant, l'actif une fois dissout étant apte à être libéré d'un côté au moins de la feuille,
- replier et assembler ladite feuille sur elle-même ou la conformer en poche ou l'assembler avec au moins une autre feuille, de manière à créer une enveloppe présentant une cavité permettant d'introduire une partie du corps.

La structure composite peut être réalisée de la manière suivante :
- enduire d'une matrice adhésive à base d'adhésif permanent une première couche, cette matrice adhésive contenant au moins un actif et éventuellement une charge, la nature et la quantité de l'actif et/ou de la charge étant choisies pour permettre la libération de l'actif lorsque la structure composite est mouillée par un solvant,
- assembler la première couche ainsi revêtue avec une deuxième couche, de telle sorte que la matrice adhésive soit prise en sandwich entre les deux couches et ces dernières liées de manière permanente directement ou indirectement par la matrice, l'une au moins des deux couches étant perméable à un solvant capable de dissoudre l'actif contenu dans la matrice. La deuxième couche peut être revêtue sur une face d'une deuxième matrice adhésive, les deux matrices adhésives pouvant alors être contrecollées. Il est avantageux de réunir deux matrices adhésives, même de compositions identiques, car cela permet d'assembler aisément deux couches extérieures différentes, en vue par exemple de disposer de deux faces offrant des caractéristiques d'application spécifiques.

On comprend qu'il est aisé de fabriquer, grâce à l'invention, de manière indépendante et en grande quantité, des couches perméables ou non revêtues chacune d'une matrice adhésive contenant un ou plusieurs actifs prédéterminés, et de réaliser ensuite de par le choix des couches que l'on assemble, des combinaisons d'actifs particulières, selon l'utilisation à laquelle est destinée le dispositif de traitement. On peut, en particulier, facilement réaliser de cette manière une structure composite comportant deux couches extérieures et deux matrices adhésives contenant des actifs devant être stockés séparément.

L'invention a encore pour objet divers procédés de traitement cosmétologique.

Au sens de la présente, un produit cosmétique est un produit tel que défini dans la Directive 93/35/CEE du Conseil du 14 juin 1993.

Un traitement cosmétique s'entend d'un traitement au moyen d'un produit tel que défini ci-dessus.

L'invention a ainsi notamment pour objet un procédé de traitement cosmétique d'une partie du corps, comportant l'étape suivante :
- introduire la partie du corps à traiter dans la cavité d'une enveloppe d'un dispositif de traitement réalisé conformément à l'invention, avant ou après la mise en contact de cette enveloppe avec le solvant.

Avantageusement, on provoque un mouvement de massage entre l'enveloppe et la partie du corps insérée à l'intérieur. Un tel massage peut favoriser la libération du ou des actifs vers l'intérieur de l'enveloppe.

Eventuellement, après un premier traitement, l'enveloppe peut être retournée et l'on peut recommencer le traitement avec l'enveloppe retournée.

Le procédé peut également comporter l'étape consistant à choisir entre deux faces différentes de l'enveloppe avant d'effectuer un premier traitement.

Un dispositif de traitement conforme à l'invention peut servir à tout traitement thérapeutique en général. Il peut notamment servir à traiter des maladies tels que l'acné, l'herpès cutané ou le psoriasis, entre autres.

L'invention a encore pour objet un procédé de traitement cosmétique d'au moins une partie d'au moins une main, notamment de la totalité des mains, comportant l'étape suivante :
- introduire la main dans un gant comportant une enveloppe telle que définie plus haut, avant ou après l'introduction du solvant.

L'invention a encore pour objet un gant destiné au traitement d'au moins une partie d'au moins une main.

Un tel gant peut comporter au moins un actif hydratant, anti-crevasse, anti-herpès, de soin des brûlures, contre l'eczéma ou favorisant la cicatrisation.

D'autres caractéristiques et avantages de la présente invention ressortiront à la lecture de la description qui va suivre, d'exemples non limitatifs de l'invention, et à l'examen du dessin annexé, qui fait partie intégrante de la description, et sur lequel :
- la figure 1 est une vue schématique de face d'un gant de traitement réalisé conformément à l'invention,
- la figure 2 est une section schématique selon II-II de la figure 1,
- la figure 3 est une vue de côté d'un doigt de gant réalisé conformément à l'invention,
- la figure 4 est une section transversale selon IV-IV de la figure 3,
- la figure 5 est une vue de côté d'une variante de réalisation du doigt de gant de la figure 3,
- la figure 6 est une coupe transversale selon VI-VI de la figure 5,
- la figure 7 représente un bonnet de traitement réalisé conformément à l'invention,
- la figure 8 est une section schématique selon VIII-VIII de la figure 7,
- la figure 9 représente une chaussette de traitement conforme à l'invention,
- la figure 10 représente une poche de traitement réalisée conformément à l'invention,
- la figure 11 représente une cagoule de traitement réalisée conformément à l'invention,
- les figures 12 à 20 représentent différents exemples de structures pouvant être utilisées,
- la figure 21 illustre la fabrication de la feuille de la figure 20,
- la figure 22 représente une variante de réalisation de la structure de la figure 20, et
- la figure 23 représente le détail XXIII-XXIII de la figure 2.

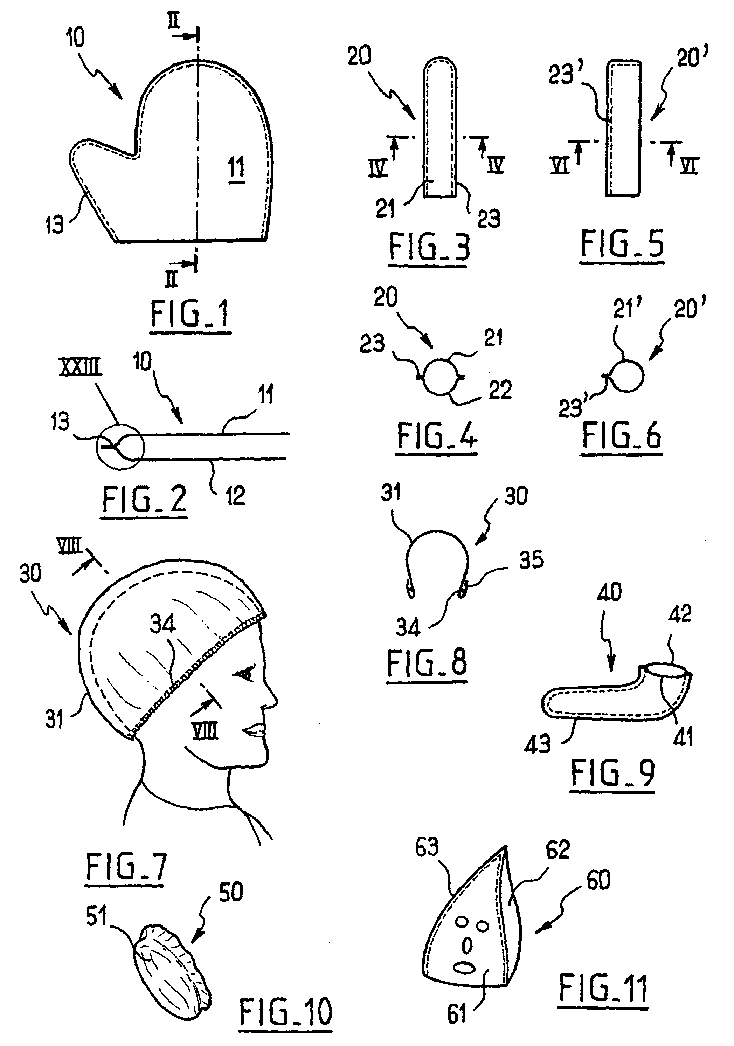

On a représenté à la figure 1 un gant de traitement 10 comportant, comme on peut le voir sur la figure 2, deux feuilles 11 et 12 assemblées à leur périphérie dans une zone d'assemblage 13 (représentée en pointillés) pour former une enveloppe définissant une cavité à l'intérieur de laquelle peut être introduite une main.

Dans l'exemple considéré, les feuilles 11 et 12 sont suffisamment souples pour permettre de retourner le gant sur lui-même afin de procéder éventuellement à un nouveau traitement, comme cela sera précisé plus loin.

Au moins l'une des feuilles 11 ou 12 comporte une matrice adhésive disposée de manière permanente entre au moins deux couches, au contact direct ou non de ces couches, cette matrice adhésive contenant au moins un actif et l'une au moins desdites couches étant perméable à un solvant tel que de l'eau par exemple, capable de dissoudre cet actif afin d'entraîner sa libération au contact de la surface à traiter.

La libération de l'actif peut avoir lieu à l'intérieur de la cavité formée par l'enveloppe, à l'extérieur de l'enveloppe ou les deux, selon les applications recherchées.

L'invention n'est pas limitée à un dispositif de traitement ayant la forme d'un gant.

On a représenté sur les figures 3 à 11 divers autres exemples de dispositifs de traitement, les exemples donnés n'étant pas limitatifs.

On a ainsi représenté à la figure 3 un doigt de gant 20 destiné au traitement d'un doigt, comprenant une enveloppe tubulaire définissant une cavité pour recevoir le doigt.

Dans l'exemple des figures 3 et 4, le doigt de gant est formé par l'assemblage de deux feuilles 21 et 22 le long d'une zone d'assemblage 23 (représentée en pointillés) à leur périphérie.

On a représenté sur les figures 5 et 6 un doigt de gant 20' conforme à une variante de réalisation, dans laquelle la cavité est formée par une seule feuille 21' repliée sur elle-même et assemblée selon une zone d'assemblage 23' (représentée en pointillés) s'étendant le long des bords longitudinaux de la feuille 21' et à l'opposé de l'ouverture d'introduction du doigt.

On a représenté à la figure 7 un bonnet de traitement 30 formé par une feuille 31 conformée en poche. Cette feuille 31 est munie à sa périphérie d'un élastique 34, lequel est maintenu par un revers 35. L'élastique 34 est assemblé à l'état tendu avec la feuille 31, de telle sorte que cette dernière soit froncée à sa périphérie au repos. Le bonnet de traitement 30 sert par exemple à l'application d'un soin sur les cheveux ou le cuir chevelu.

On a représenté à la figure 9 un chausson de traitement 40 formé par l'assemblage à leur périphérie de deux feuilles 41 et 42 le long d'une zone d'assemblage 43 (représentée en pointillés).

On a représenté à la figure 10 une poche de traitement 50, formée de manière similaire au bonnet de traitement de la figure 7 avec une feuille unique 51, mais de taille inférieure, par exemple adaptée à contenir une oreille.

On a représenté à la figure 11 une cagoule de traitement 60 formée par l'assemblage à leur périphérie de deux feuilles 61 et 62 le long d'une zone d'assemblage 63 (représentée en pointillés).

On va maintenant décrire divers exemples de structures composites possibles pour la ou les feuilles servant à réaliser l'enveloppe de chacun des dispositifs de traitement qui viennent d'être décrits.

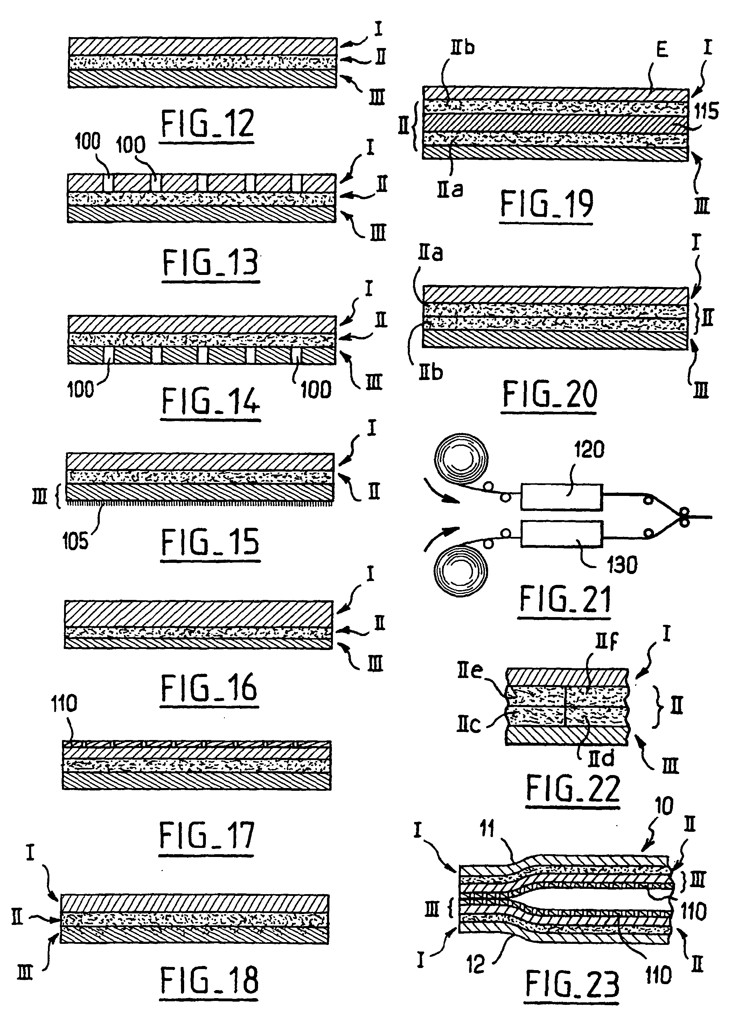

On a représenté sur la figure 12 une structure composite conforme à un exemple de mise en oeuvre de l'invention. Cette structure composite comporte une matrice adhésive II prise en sandwich entre deux couches I et III. L'une au moins de ces couches I et III est perméable à un solvant, par exemple de l'eau, une huile ou une lotion, notamment une lotion alcoolique.

La matrice adhésive II est à base d'adhésif permanent, non soluble dans ledit solvant, et permet d'assurer la cohésion des deux couches I et III même lorsque la structure composite est mouillée. La matrice adhésive II contient par exemple au moins un actif hydrosoluble pour le nettoyage, le maquillage ou le soin de la peau ou des cheveux et une charge destinée à lui permettre de relarguer le ou les actifs qu'elle contient lorsque la structure composite est imprégnée de solvant. La matrice adhésive II peut être à base vinylique, à base de PVA ou PVP, de pseudo latex tels que les polymères acryliques, de polyuréthanes, d'élastomères de latex, cette liste étant non limitative. L'adhésif choisi peut être réversible (cas par exemple du PVA ou du PVP) ou non (cas par exemple des acryliques, vinyliques, polyuréthanes et élastomères de latex).

Dans un exemple particulier où le solvant est de l'eau, la matrice adhésive II peut comporter une charge capable de lui permettre d'absorber l'eau, de telle sorte qu'elle perde de sa cohésion et que le ou les actifs hydrosolubles qu'elle contient soient libérés plus facilement lorsque la structure composite est mouillée. Comme charge, on peut utiliser des particules d'un hydroabsorbant tel que par exemple un polyacrylate.

On peut incorporer d'une manière générale dans la matrice adhésive II, par exemple entre 0,01 et 50 % d'actifs, choisis par exemple dans la liste suivante : vitamine C, vitamine A, vitamine F, laponite, glycérine, tensioactifs, collagène, acide salicylique, huiles essentielles aromatiques, colorants, caféine. On peut incorporer également dans la matrice adhésive une charge pulvérulente d'un matériau inerte, par exemple de l'ORGASOL.

Pour constituer les couches I et III, on peut notamment utiliser divers composants souples perméables, extensibles ou non, tel qu'un film textile, un non-tissé, un matériau alvéolaire tels qu'une mousse, un feutre, ou tout stratifié comportant l'un de ces composants laminé avec un ou plusieurs autres composants appartenant à cette liste ou non. L'une des couches I ou III peut encore comporter un ou plusieurs composants souples imperméables ou partiellement occlusifs, par exemple un film plastique, métallisé ou non, ou une feuille de métal. Les couches I et III peuvent être intrinsèquement, de par la nature du ou des matériaux les constituant, hydrophiles ou hydrophobes, ou avoir reçu un traitement destiné à les rendre hydrophiles ou hydrophobes. Les couches I et III peuvent être d'épaisseurs différentes, de natures différentes, de couleurs différentes, de rugosités différentes, entre autres.

L'une au moins des couches I et III peut comporter des perforations ou fentes 100, comme illustré sur les figures 13 et 14. Ces perforations ou fentes 100 peuvent favoriser la diffusion des actifs contenus dans la matrice adhésive vers la peau, et déboucher vers l'extérieur ou l'intérieur de l'enveloppe. Il convient de noter que les perforations ou fentes réalisées peuvent être suffisamment étroites pour empêcher la matrice adhésive de venir directement au contact de la peau. Les perforations ou fentes 100 peuvent contribuer à rendre l'enveloppe extensible.

L'une au moins des couches I ou III peut être revêtue d'un flocage 105, comme illustré à la figure 15.

La structure composite peut comporter une matrice adhésive II prise en sandwich entre des couches I et III d'épaisseurs différentes, comme illustré à la figure 16, ces couches extérieures I et III étant constituées par exemple par des non-tissés de textures différentes, l'une douce et l'autre plus rugueuse. L'utilisateur a ainsi le choix au moment de l'utilisation entre deux types de surfaces, selon par exemple qu'il souhaite désincruster des impuretés de la surface de la peau ou effectuer un simple nettoyage superficiel, le choix de la surface s'effectuant en retournant l'enveloppe sur elle-même par exemple.

L'une des couches I et III de la feuille peut comporter un voile 110 en surface, destiné à faciliter l'assemblage avec une autre feuille, notamment par soudure, comme illustré à la figure 17. Ce voile 110 peut être laminé avec un non-tissé, par exemple.

Afin de favoriser la diffusion d'actifs vers une face de l'enveloppe, la matrice peut imprégner l'une des couches I et III sur une partie au moins de son épaisseur, comme illustré sur la figure 18. Une telle imprégnation s'obtient par exemple en déposant la matrice à l'état fluide sur l'une des couches I et III, de manière à lui permettre de diffuser dans cette couche.

Plusieurs matrices adhésives peuvent être superposées directement ou indirectement, ce qui permet de réaliser un grand nombre de combinaisons d'actifs et/ou de couches de support ayant des propriétés différentes. Les matrices adhésives peuvent être assemblées de diverses manières, étant par exemple contrecollées ou prises individuellement en sandwich entre des couches perméables ou imperméables.

A titre d'exemple, on a représenté sur la figure 19 une structure composite comportant un sous-ensemble II pris en sandwich entre des couches extérieures I et III. Le sous-ensemble II est constitué par deux matrices adhésives IIa et IIb disposées de part et d'autre d'une couche intermédiaire 115. Les matrices adhésives IIa et IIb comprennent par exemple des actifs différents, et l'utilisateur peut ainsi traiter la peau de manière différente, selon que la matrice adhésive IIa est située du côté intérieur ou extérieur de l'enveloppe.

La couche intermédiaire 115 peut être imperméable, de sorte que les actifs contenus dans la matrice IIa ne diffusent pas vers la matrice IIb et réciproquement, ou perméable.

On a représenté sur la figure 20 une structure composite dans laquelle la matrice adhésive II est constituée de deux sous-matrices IIa et IIb contrecollées, prises en sandwich entre deux couches I et III. L'une de ces couches I et III peut être occlusive, et se situer du côté intérieur ou extérieur de l'enveloppe.

Les matrices adhésives IIa et IIb comportent par exemple des actifs différents, notamment des actifs ne pouvant pas être conditionnés ensemble.

Pour réaliser la structure composite représentée à la figure 20, on peut partir comme illustré sur la figure 21 des couches I et III sur lesquelles on dépose, à des postes d'enduction 120 et 130 connus en eux-mêmes, les matrices adhésives IIa et IIb. Ces dernières peuvent contenir des solvants lors de la fabrication, afin de faciliter l'opération d'enduction. Ces solvants sont volatils et destinés à être éliminés de la structure composite finale. Les couches I et III ainsi revêtues de leurs matrices adhésives respectives sont ensuite contrecollées pour former la structure composite finale.

On comprend que l'on peut grâce à l'invention réaliser de manière indépendante une pluralité de couches imprégnées chacune d'une matrice adhésive contenant des actifs prédéterminés, et assembler les couches de support ainsi revêtues de manière à obtenir la combinaison d'actifs recherchée.

On a représenté sur la figure 22 une matrice adhésive comportant plusieurs régions IIc, IId, IIe et IIf contenant des actifs différents. Cette configuration peut être utilisée notamment lorsqu'il est nécessaire de conditionner au sein d'une même matrice adhésive plusieurs actifs devant être stockés séparément. La configuration de la figure 22 peut également être utile pour multiplier le nombre d'actifs stockés séparément dans une feuille.

La région IIc peut contenir par exemple de la vitamine C, la région IId une enzyme, la région IIe du bicarbonate et la région IIf de l'acide citrique. Ces différents composés, une fois libérés après mise au contact de l'enveloppe avec un solvant tel que de l'eau, permettent de former du rétinol moussant pour traiter des signes de vieillissement.

A titre d'exemple, on a réalisé un gant de nettoyage tel que représenté à la figure 1, ayant une structure conforme à celle illustrée à la figure 23, la couche III située du côté intérieur du gant étant constituée par un non-tissé de polyethylène laminé avec un voile thermosoudable. La matrice adhésive II est à base d'adhésif permanent polyacrylique contenant de la glycérine. La couche extérieure I est constituée d'un non-tissé. Lors de l'utilisation, la main est introduite dans le gant puis de l'eau est versée à l'intérieur. La main est fermée sur elle-même à plusieurs reprises. Une fois la main extraite, on la laisse sécher. La main est non grasse et donne pendant quelques heures la sensation d'être moins moite que l'autre main, non traitée.

Bien entendu, l'invention n'est pas limitée aux exemples qui viennent d'être décrits.

En particulier, on peut réaliser d'autres combinaisons de couches et de matrices adhésives.

La matrice adhésive peut encore être utilisée à la manière d'un réservoir d'actifs et la structure composite mouillée plusieurs fois de suite.

On peut utiliser un autre solvant que de l'eau pour mouiller la structure composite, ce solvant devant bien entendu être compatible avec un usage externe sur l'utilisateur.

## Revendications

1. Dispositif de traitement (10 ; 20 ; 20' ; 30 ; 40 ; 50 ; 60) comportant une enveloppe définissant une cavité dans laquelle peut être engagée une partie du corps, cette enveloppe comportant au moins une feuille (11, 12) présentant une structure composite **caractérisé par le fait que** la structure composite comporte au moins une matrice adhésive (II) située entre deux couches (I ; III) dont l'une au moins est perméable à un solvant, ces deux couches étant liées de manière permanente à la matrice adhésive, cette dernière comprenant au moins un actif soluble dans ledit solvant, l'actif une fois dissout étant apte à être libéré au moins d'un côté de l'enveloppe lorsque le traitement a lieu et après avoir mis toute ou partie de l'enveloppe au contact du solvant.

2. Dispositif selon la revendication 1, **caractérisé par le fait que** l'enveloppe (10) présente la forme d'un gant.

3. Dispositif selon la revendication 1, **caractérisé par le fait que** l'enveloppe (30) présente la forme d'un bonnet, d'un doigt de gant, d'une cagoule, d'un chausson ou d'une poche à disposer autour d'une oreille.

4. Dispositif selon l'une quelconque des revendications 1 à 3, **caractérisé par le fait que** l'enveloppe est élastiquée.

5. Dispositif selon l'une quelconque des revendications 1 à 4, **caractérisé par le fait que** l'enveloppe est réalisée à partir d'une feuille unique repliée sur elle-même.

6. Dispositif selon l'une quelconque des revendications 1 à 4, **caractérisé par le fait que** l'enveloppe est réalisée à partir d'une feuille unique conformée en poche.

7. Dispositif selon l'une quelconque des revendications 1 à 4, **caractérisé par le fait que** l'enveloppe est réalisée à partir de deux ou plusieurs feuilles assemblées entres elles, par exemple le long d'une zone (13 ; 23 ; 63) située en périphérie.

8. Dispositif selon la revendication 7, **caractérisé par le fait que** les feuilles sont assemblées par soudure, collage ou couture.

9. Dispositif selon l'une quelconque des revendications précédentes, **caractérisé par le fait que** la matrice adhésive (II) contient un ou plusieurs actifs solubles dans le solvant utilisé et/ou capables de gonfler au contact dudit solvant, dans une quantité suffisante pour que la matrice perde de sa cohésion au contact du solvant et libère plus facilement le ou les actifs.

10. Dispositif selon l'une quelconque des revendications précédentes, **caractérisé par le fait que** la matrice adhésive (II) contient une charge d'un ou plusieurs composés sensiblement inertes, dans une quantité suffisante pour que la matrice perde de sa cohésion au contact du solvant et libère plus facilement le ou les actifs.

11. Dispositif selon l'une quelconque des revendications précédentes, **caractérisé par le fait que** le solvant utilisé est de l'eau.

12. Dispositif selon l'une quelconque des revendications 1 à 10, **caractérisé par le fait que** le solvant utilisé est une huile.

13. Dispositif selon l'une quelconque des revendications 1 à 10, **caractérisé par le fait que** le solvant utilisé est une lotion.

14. Dispositif selon l'une quelconque des revendications précédentes, **caractérisé par le fait que** la matrice adhésive (II) comporte un ou plusieurs composés absorbeurs d'humidité, notamment entre 0,2 et 60 % en masse d'un composé absorbeur d'humidité, voire entre 0,5 et 40 %, ce composé étant choisi parmi les polyacrylates, les silices, les fibres de coton, les amidons, les alginates, les carbonates de calcium ou de magnésium, la viscose, la cellulose, les lyophylisats.

15. Dispositif selon l'une quelconque des revendications précédentes, **caractérisé par le fait que** la matrice adhésive (II) comporte une ou plusieurs substances capables d'abaisser son pouvoir adhésif et lui permettre d'éclater au contact du solvant afin de faciliter la libération du ou des actifs, notamment des substances sensiblement inertes telles que des microbilles ou une poudre d'un composé inerte, notamment une poudre de polyamide connue sous la dénomination ORGASOL.

16. Dispositif selon l'une quelconque des revendications précédentes, **caractérisé par le fait que** la matrice adhésive (II) comporte un ou plusieurs actifs choisis dans la liste suivante : glycérine, colorants, hydratants, adoucissants, antirides, rafraîchissants, anti-perspirants et nourrissants.

17. Dispositif selon l'une quelconque des revendications précédentes, **caractérisé par le fait que** la matrice adhésive comporte un ou plusieurs actifs choisis dans la liste suivante : vitamine C, vitamine A, vitamine F, laponite, tensioactifs, collagène, acide salicylique, acide tio, huiles essentielles aromatiques, caféine, anti-oxydants, anti-radicaux libres, dépigmentants, liporégulateurs, anti-acnéiques, anti-séborrhéiques, anti-vieillissement, kératolitiques, anti-inflammatoires, cicatrisants, protecteurs vasculaires, anti-bactériens, anti-fongiques, conditionneurs de la peau, insensibilisants et immunomodulateurs.

18. Dispositif selon l'une quelconque des revendications précédentes, **caractérisé par le fait que** la matrice adhésive comporte des particules magnétisables ou magnétiques.

19. Dispositif selon l'une quelconque des revendications précédentes, **caractérisé par le fait que** l'une au moins des couches (I ; III) au contact de la matrice adhésive est constituée par un non-tissé.

20. Dispositif selon l'une quelconque des revendications précédentes, **caractérisé par le fait que** la matrice adhésive (II) est située entre deux couches (I ; III) de non-tissé.

21. Dispositif selon l'une quelconque des revendications précédentes, **caractérisé par le fait que** les faces intérieure ou extérieure de l'enveloppe présentent des rugosités différentes.

22. Dispositif selon l'une quelconque des revendications précédentes, **caractérisé par le fait que** les couches (I ; III) présentent des porosités différentes.

23. Dispositif selon l'une quelconque des revendications précédentes, **caractérisé par le fait que** les couches (I ; III) présentent des couleurs différentes.

24. Dispositif selon l'une quelconque des revendications précédentes, **caractérisé par le fait que** les couches (I ; III) présentent des épaisseurs différentes.

25. Dispositif selon l'une quelconque des revendications précédentes, **caractérisé par le fait que** l'enveloppe présente sur une face un voile thermosoudable (110).

26. Dispositif selon la revendication 25, **caractérisé par le fait que** ledit voile est présent sur une face d'un non-tissé lié par l'autre face à la matrice adhésive.

27. Dispositif selon l'une quelconque des revendications précédentes, **caractérisé par le fait que** la structure composite comporte une couche imperméable (115) ou partiellement imperméable.

28. Dispositif selon la revendication 27, **caractérisé par le fait que** la couche imperméable ou partiellement imperméable est présente entre la matrice et l'extérieur de l'enveloppe.

29. Dispositif selon l'une quelconque des revendications précédentes, **caractérisé par le fait que** la structure composite comporte au moins deux matrices adhésives (IIa, IIb, IIc, IId, IIe, IIf) juxtaposées ou superposées, de compositions identiques ou différentes.

30. Dispositif selon l'une des revendications 1 à 18, **caractérisé par le fait que** la structure composite comporte, dans l'ordre, la superposition de couches suivante : une première couche (I) perméable au solvant, une première matrice adhésive (IIa), une deuxième matrice adhésive (IIb) et une deuxième couche (III) perméable au solvant.

31. Dispositif selon l'une des revendications 1 à 18, **caractérisé par le fait que** la structure composite comporte, dans l'ordre, la superposition de couches suivante : une première couche perméable (I), une première matrice adhésive (IIb) contenant au moins un actif, une couche intermédiaire (115) imperméable, une deuxième matrice adhésive (IIa) contenant au moins un actif, et une deuxième couche perméable (III), les première et deuxième matrices adhésives contenant des actifs différents.

32. Dispositif selon l'une quelconque des revendications précédentes, **caractérisé par le fait que** la feuille (11, 12) est agencée pour permettre une diffusion préférentielle de l'actif vers un côté de l'enveloppe.

33. Dispositif selon la revendication 32, **caractérisé par le fait que** la feuille est agencée pour permettre une diffusion préférentielle de l'actif vers l'intérieur de l'enveloppe.

34. Procédé de fabrication d'un dispositif de traitement d'une partie du corps selon l'une quelconque des revendications précédentes, comportant les étapes suivantes :
- fournir au moins une feuille (11, 12 ; 21, 22 ; 21' ; 31 ; 41, 42 ; 61, 62) comprenant une structure composite comportant au moins une matrice adhésive (II) présente entre deux couches (I ; III) dont l'une au moins est perméable à un solvant, ces deux couches étant liées de manière permanente à la matrice adhésive, cette dernière comprenant au moins un actif soluble dans le solvant, l'actif une fois dissout étant apte à être libéré d'un côté au moins de la feuille,
- replier et assembler ladite feuille sur elle-même ou la conformer en poche ou l'assembler avec au moins une autre feuille de manière à créer une enveloppe présentant une cavité permettant d'introduire une partie du corps.

35. Procédé de traitement cosmétique d'une partie du corps, comportant l'étape suivante :
- introduire la partie du corps à traiter dans la cavité de l'enveloppe d'un dispositif de traitement réalisé conformément à l'une quelconque des revendications 1 à 33 sauf la revendication 17, avant ou après l'introduction du solvant.

36. Procédé selon la revendication 35, **caractérisé par le fait que** l'on provoque un mouvement de massage entre l'enveloppe et la partie du corps insérée à l'intérieur.

37. Procédé selon la revendication 35 ou 36, **caractérisé par le fait que**, après un premier traitement, l'enveloppe est retournée et l'on recommence le traitement avec l'enveloppe retournée.

38. Gant destiné au traitement des mains, constitué par l'enveloppe d'un dispositif tel que défini dans l'une quelconque des revendications 1 et 2 et 4 à 33.

39. Gant selon la revendication précédente, **caractérisé par le fait qu'**il comporte au moins un actif hydratant.

40. Gant selon la revendication 39, **caractérisé par le fait qu'**il comporte au moins un actif anti-crevasse, anti-herpès, de soin des brûlures, contre l'eczéma ou favorisant la cicatrisation

41. Procédé de traitement cosmétique des mains comportant l'étape suivante :
- introduire la main dans un gant tel que défini dans l'une des revendications 38 et 39 sans rattachement à la revendication 17, avant ou après l'introduction du solvant.

## Patentansprüche

1. Vorrichtung (10; 20; 20'; 30; 40; 50; 60) zur kosmetischen oder dermatologischen Behandlung, umfassend eine Hülle, die einen Hohlraum begrenzt, in den ein Teil des Körpers eingeführt werden kann, wobei diese Hülle mindestens eine Folie (11, 12) umfaßt, die eine Verbundstruktur besitzt, **dadurch gekennzeichnet, daß** die Verbundstruktur mindestens eine haftende Matrix (II) aufweist, die zwischen zwei Schichten (I; III) gelegen ist, von denen mindestens eine für ein Lösungsmittel durchlässig ist, wobei diese beiden Schichten mit der haftenden Matrix bleibend verbunden sind, wobei letztere mindestens einen in diesem Lösungsmittel lösbaren Wirkstoff enthält, wobei der Wirkstoff im gelösten Zustand auf mindestens einer Seite der Hülle freigesetzt werden kann, wenn die Behandlung stattfindet und nachdem die Hülle ganz oder teilweise mit dem Lösungsmittel in Kontakt gebracht wurde.

2. Vorrichtung nach Anspruch 1, **dadurch gekennzeichnet, daß** die Hülle (10) die Form eines Handschuhs hat.

3. Vorrichtung nach Anspruch 1, **dadurch gekennzeichnet, daß** die Hülle (30) die Form einer Haube, eines Handschuhfingers, einer Kapuzenmaske, eines Schuhs oder einer um das Ohr herum anzuordnenden Tasche hat.

4. Vorrichtung nach einem der Ansprüche 1 bis 3, **dadurch gekennzeichnet, daß** die Hülle elastisch ist.

5. Vorrichtung nach einem der Ansprüche 1 bis 4, **dadurch gekennzeichnet, daß** die Hülle aus einer einzigen zusammengefalteten Folie hergestellt ist.

6. Vorrichtung nach einem der Ansprüche 1 bis 4, **dadurch gekennzeichnet, daß** die Hülle aus einer einzigen, zu einer Tasche geformten Folie hergestellt ist.

7. Vorrichtung nach einem der Ansprüche 1 bis 4, **dadurch gekennzeichnet, daß** die Hülle aus zwei oder mehreren Folien herstellt ist, die beispielsweise längs einer am Umfang gelegenen Zone (13; 23; 63) zusammengefügt sind.

8. Vorrichtung nach Anspruch 7, **dadurch gekennzeichnet, daß** die Folien durch Verschweißen, Verkleben oder Vernähen zusammengefügt sind.

9. Vorrichtung nach einem der vorhergehenden Ansprüche, **dadurch gekennzeichnet, daß** die haftende Matrix (II) einen oder mehrere Wirkstoffe enthält, die in dem verwendeten Lösungsmittel löslich sind und/oder im Kontakt mit dem Lösungsmittel in einem so starken Maß quellen können, daß die Matrix im Kontakt mit dem Lösungsmittel ihre Kohäsion verliert und den oder die Wirkstoffe leichter freisetzt.

10. Vorrichtung nach einem der vorhergehenden Ansprüche, **dadurch gekennzeichnet, daß** die haftende Matrix (II) eine Charge von einer oder mehreren im wesentlichen inerten Verbindungen in einer so großen Menge enthält, daß die Matrix im Kontakt mit dem Lösungsmittel ihre Kohäsion verliert und den oder die Wirkstoffe leichter freisetzt.

11. Vorrichtung nach einem der vorhergehenden Ansprüche, **dadurch gekennzeichnet, daß** das verwendete Lösungsmittel Wasser ist.

12. Vorrichtung nach einem der Ansprüche 1 bis 10, **dadurch gekennzeichnet, daß** das verwendete Lösungsmittel ein Öl ist.

13. Vorrichtung nach einem der Ansprüche 1 bis 10, **dadurch gekennzeichnet, daß** das verwendete Lösungsmittel eine Lotion ist.

14. Vorrichtung nach einem der vorhergehenden Ansprüche, **dadurch gekennzeichnet, daß** die haftende Matrix (II) eine oder mehrere Feuchtigkeit absorbierende Verbindungen enthält, insbesondere zwischen 0,2 und 60 Massen-% einer Feuchtigkeit absorbierenden Verbindung, oder zwischen 0,5 und 40%, wobei diese Verbindung aus Polyacrylaten, Siliciumoxiden, Baumwollfasern, Stärken, Alginaten, Calcium- oder Magnesiumcarbonaten, Viscose, Cellulose, Lyophilisaten ausgewählt ist.

15. Vorrichtung nach einem der vorhergehenden Ansprüche, **dadurch gekennzeichnet, daß** die haftende Matrix (II) eine oder mehrere Substanzen enthält, die ihr Haftvermögen senken können und ihr gestatten, im Kontakt mit dem Lösungsmittel zu platzen, um die Freisetzung des oder der Wirkstoffe zu erleichtern, insbesondere im wesentlichen inerte Substanzen, wie Mikrokugeln oder ein Pulver einer inerten Verbindung, insbesondere ein unter der Bezeichnung ORGASOL bekanntes Polyamidpulver.

16. Vorrichtung nach einem der vorhergehenden Ansprüche, **dadurch gekennzeichnet, daß** die haftende Matrix (II) einen oder mehrere Wirkstoffe enthält, die aus der folgenden Liste ausgewählt sind: Glycerin, Farbstoffe, hydratisierende Mittel, weichmachende Mittel, Anti-Falten-Mittel, erfrischende Mittel, schweißhemmende Mittel und nährende Mittel.

17. Vorrichtung nach einem der vorhergehenden Ansprüche, **dadurch gekennzeichnet, daß** die haftende Matrix einen oder mehrere Wirkstoffe enthält, die aus der folgenden Liste ausgewählt sind: Vitamin C, Vitamin A, Vitamin F, Lapotin, Netzmittel, Collagen, Salicylsäure, Tiosäure, aromatische essentielle Öle, Cafein, Antioxidationsmittel, Mittel gegen freie Radikale, Entfärbungsmittel, Liporegulatoren, Mittel gegen Akne, Mittel gegen Seborrhoe, Mittel gegen Altern, keratolytisch wirkende Mittel, entzündungshemmende Mittel, wundheilende Mittel, Gefäßschutzmittel, antibakterielle Mittel, antifungische Mittel, die Haut konditionierende Mittel, desensibilisierende Mittel und immunomodulierende Mittel.

18. Vorrichtung nach einem der vorhergehenden Ansprüche, **dadurch gekennzeichnet, daß** die haftende Matrix magnetisierbare oder magnetische Teilchen enthält.

19. Vorrichtung nach einem der vorhergehenden Ansprüche, **dadurch gekennzeichnet, daß** mindestens eine der mit der Matrix in Kontakt befindlichen Schichten (I; III) aus einem Vlies besteht.

20. Vorrichtung nach einem der vorhergehenden Ansprüche, **dadurch gekennzeichnet, daß** die haftende Matrix (11) zwischen zwei Vliesschichten (I; III) gelegen ist.

21. Vorrichtung nach einem der vorhergehenden Ansprüche, **dadurch gekennzeichnet, daß** die Innen- oder Außenseiten der Hülle verschiedene Rauheiten haben.

22. Vorrichtung nach einem der vorhergehenden Ansprüche, **dadurch gekennzeichnet, daß** die Schichten (I; III) verschiedene Porositäten haben.

23. Vorrichtung nach einem der vorhergehenden Ansprüche, **dadurch gekennzeichnet, daß** die Schichten (I; III) verschiedene Farben haben.

24. Vorrichtung nach einem der vorhergehenden Ansprüche, **dadurch gekennzeichnet, daß** die Schichten (I; III) verschiedene Dicken haben.

25. Vorrichtung nach einem der vorhergehenden Ansprüche, **dadurch gekennzeichnet, daß** die Hülle auf einer Seite eine heißsiegelbare Beschichtung (110) aufweist.

26. Vorrichtung nach Anspruch 25, **dadurch gekennzeichnet, daß** diese Beschichtung auf einer Seite eines Vlieses vorgesehen ist, das über die andere Seite mit der haftenden Matrix verbunden ist.

27. Vorrichtung nach einem der vorhergehenden Ansprüche, **dadurch gekennzeichnet, daß** die Verbundstruktur eine undurchlässige oder partiell undurchlässige Schicht (115) aufweist.

28. Vorrichtung nach Anspruch 27, **dadurch gekennzeichnet, daß** die undurchlässige oder partiell undurchlässige Schicht zwischen der Matrix und dem Äußeren der Hülle vorgesehen ist.

29. Vorrichtung nach einem der vorhergehenden Ansprüche, **dadurch gekennzeichnet, daß** die Verbundstruktur mindestens zwei nebeneinander oder übereinander angeordnete haftende Matrizes (IIa, IIb IIc, IId, IIe, IIf) mit gleichen oder verschiedenen Zusammensetzungen aufweist.

30. Vorrichtung nach einem der Ansprüche 1 bis 18, **dadurch gekennzeichnet, daß** die Verbundstruktur die folgende Überlagerung von Schichten in dieser Reihenfolge aufweist: eine erste für das Lösungsmittel durchlässige Schicht (I), eine erste haftende Matrix (IIa), eine zweite haftende Matrix (IIb) und eine zweite für das Lösungsmittel durchlässige Schicht (III).

31. Vorrichtung nach einem der Ansprüche 1 bis 18, **dadurch gekennzeichnet, daß** die Verbundstruktur die folgende Überlagerung von Schichten in dieser Reihenfolge aufweist: eine erste durchlässige Schicht (I), eine erste haftende Matrix (IIb), die mindestens einen Wirkstoff enthält, eine undurchlässige Zwischenschicht (115), eine zweite haftende Matrix (IIa), die mindestens einen Wirkstoff enthält, und eine zweite durchlässige Schicht (III), wobei die erste und die zweite haftende Matrix verschiedene Wirkstoffe enthalten.

32. Vorrichtung nach einem der vorhergehenden Ansprüche, **dadurch gekennzeichnet, daß** die Folie (11, 12) ausgebildet ist, um eine bevorzugte Diffusion des Wirkstoffs auf eine Seite der Hülle zu zu gestatten.

33. Vorrichtung nach Anspruch 32, **dadurch gekennzeichnet, daß** die Folie ausgebildet ist, um eine bevorzugte Diffusion des Wirkstoffs auf das Innere der Hülle zu zu gestatten.

34. Verfahren zur Herstellung einer Vorrichtung zur Behandlung eines Teils des Körpers nach einem der vorhergehenden Ansprüche, umfassend die folgenden Schritte:
- Liefern mindestens einer Folie (11, 12; 21, 22; 21'; 31; 41, 42; 61, 62), die eine Verbundstruktur besitzt, die mindestens eine haftende Matrix (II) umfaßt, die zwischen zwei Schichten (I; III) vorgesehen ist, von denen mindestens eine für ein Lösungsmittel durchlässig ist, wobei diese beiden Schichten mit der haftenden Matrix bleibend verbunden sind, wobei letztere mindestens einen in dem Lösungsmittel löslichen Wirkstoff enthält, wobei der Wirkstoff im gelösten Zustand auf mindestens einer Seite der Folie freigesetzt werden kann,
- Zusammenfalten und Zusammenfügen der Folie oder Formen der Folie zu einer Tasche oder Zusammenfügen der Folie mit mindestens einer anderen Folie, so daß eine Hülle geschaffen wird, die einen Hohlraum aufweist, der die Einführung eines Teils des Körpers gestattet.

35. Verfahren zur kosmetischen Behandlung eines Teils des Körpers, umfassend den folgenden Schritt:
- Einführen des zu behandelnden Teils des Körpers in den Hohlraum der Hülle einer gemäß einem der Ansprüche 1 bis 33, ausgenommen Anspruch 17, ausgebildeten Behandlungsvorrichtung vor oder nach der Einführung des Lösungsmittels.

36. Verfahren nach Anspruch 35, **dadurch gekennzeichnet, daß** man zwischen der Hülle und dem in ihr Inneres eingeführten Teil des Körpers eine Massagebewegung erzeugt.

37. Verfahren nach Anspruch 35 oder 36, **dadurch gekennzeichnet, daß** die Hülle nach einer ersten Behandlung gewendet wird und die Behandlung mit der gewendeten Hülle wieder begonnen wird.

38. Handschuh zur Behandlung der Hände, bestehend aus der Hülle einer Vorrichtung, wie sie in einem der Ansprüche 1 und 2 und 4 bis 33 definiert ist.

39. Handschuh nach dem vorhergehenden Anspruch, **dadurch gekennzeichnet, daß** er mindestens ein hydratisierendes Mittel enthält.

40. Handschuh nach Anspruch 39, **dadurch gekennzeichnet, daß** er mindestens ein Mittel gegen Schrunden, ein Mittel gegen Herpes, ein Pflegemittel für Verbrennungen, ein Mittel gegen Ekzeme oder ein die Wundheilung förderndes Mittel enthält.

41. Verfahren zur kosmetischen Behandlung der Hände, umfassend den folgenden Schritt:
- Einführen der Hand in einen Handschuh, wie er in einem der Ansprüche 38 und 39 ohne die Rückbeziehung auf den Anspruch 17 definiert ist, vor oder nach der Einführung des Lösungsmittels.

## Claims

1. A cosmetic or dermatologic treatment device (10; 20; 20'; 30; 40; 50; 60) comprising a cover defining a cavity into which a part of the body can be engaged, said cover comprising at least one sheet (11, 12) presenting a composite structure **characterized by** the fact that the composite structure comprises at least one adhesive matrix (II), situated between two layers (I, III), at least one of which is permeable to a solvent, the two layers being bonded in permanent manner to the adhesive matrix, the adhesive matrix containing at least one active agent that is soluble in said solvent, with the active agent, once dissolved, being suitable for being released from at least one side of the cover when the treatment is performed and after having put all or part of the cover in contact with the solvent.

2. A device according to claim 1, **characterized by** the fact that the cover (10) is in the form of a glove.

3. A device according to claim 1, **characterized by** the fact that the cover (30) is in the form of a cap, a glove finger, a hood, a sock, or a bag for placing around an ear.

4. A device according to any one of claims 1 to 3, **characterized by** the fact that the cover is elasticized.

5. A device according to any one of claims 1 to 4, **characterized by** the fact that the cover is made from a single sheet folded onto itself.

6. A device according to any one of claims 1 to 4, **characterized by** the fact that the cover is made from a single sheet shaped to form a bag.

7. A device according to any one of claims 1 to 4, **characterized by** the fact that the cover is made from two or more sheets that are assembled together, e.g. along a zone (13; 23; 63) situated at the periphery.

8. A device according to claim 7, **characterized by** the fact that the sheets are assembled together by heat-sealing, adhesive, or stitching.

9. A device according to any one of the preceding claims, **characterized by** the fact that the adhesive matrix (II) contains one or more active agents soluble in the solvent used and/or capable of swelling in contact with said solvent, and in sufficient quantity for the matrix to lose its cohesion on contact with the solvent and release the active agent(s) more easily.

10. A device according to any one of the preceding claims, **characterized by** the fact that the adhesive matrix (II) contains a filler of one or more substantially inert compounds, in sufficient quantity for the matrix to lose its cohesion on contact with the solvent and release the active agent(s) more easily.

11. A device according to any one of the preceding claims, **characterized by** the fact that the solvent used is water.

12. A device according to any one of claims 1 to 10, **characterized by** the fact that the solvent used is an oil.

13. A device according to any one of claims 1 to 10, **characterized by** the fact that the solvent used is a lotion.

14. A device according to any one of the preceding claims, **characterized by** the fact that the adhesive matrix (II) contains one or more moisture-absorbing compounds, in particular 0.2% to 60% by weight of a moisture-absorbing compound, or indeed 0.5% to 40%, said compound being selected from polyacrylates, silicas, cotton fibers, starches, alginates, calcium carbonate, magnesium carbonate, viscose, cellulose, and lyophylisates.

15. A device according to any one of the preceding claims, **characterized by** the fact that the adhesive matrix (II) contains one or more substances capable of lowering its adhesive power and enabling it to burst in contact with the solvent so as to facilitate release of the active agent(s), in particular substances that are substantially inert such as microbeads or a powder of an inert compound, in particular a polyamide powder known under the name ORGASOL.

16. A device according to any one of the preceding claims, **characterized by** the fact that the adhesive matrix (II) comprised one or more active agents selected from the followings list: glycerin, coloring agents, moisturizers, softeners, antiwrinkle agents, fresheners, antiperspirants and nourishing agents.

17. A device according to any one of the preceding claims, **characterized by** the fact that the adhesive matrix contains one or more active agents selected from the following list: vitamin C, vitamin A, vitamin F, laponite, wetting agents, collagen, salicylic acid, tio acid, essential aromatic oils, caffeine, anti-oxidants, free radical scavengers, depigmenting agents, liporegulators, anti-acne agents, antidandruff agents, anti-aging agents, keratolitic agents, anti-inflammatory agents, healing agents, vascular protectors, antibacterial agents, antifungal agents, skin conditioners, anesthetics and immunomodulators.

18. A device according to any one of the preceding claims, **characterized by** the fact that the adhesive matrix contains particles that are magnetic or magnetizable.

19. A device according to any one of the preceding claims, **characterized by** the fact that at least one of the layers (I; II) in contact with the adhesive matrix is constituted by a non-woven fabric.

20. A device according to any one of the preceding claims, **characterized by** the fact that the adhesive matrix (II) is situated between two layers (I, III) of non-woven fabric.

21. A device according to any one of the preceding claims, **characterized by** the fact that the inside and outside faces of the cover present different roughnesses.

22. A device according to any one of the preceding claims, **characterized by** the fact that the layers (I; III) present different porosities.

23. A device according to any one of the preceding claims, **characterized by** the fact that the layers (I; III) present different colors.

24. A device according to any one of the preceding claims, **characterized by** the fact that the layers (I; III) present different thicknesses.

25. A device according to any one of the preceding claims, **characterized by** the fact that the cover has a heat-sealable web (110) on one face.

26. A device according to claim 25, **characterized by** the fact that said web is present on a face of a non-woven fabric bonded via its other face to the adhesive matrix.

27. A device according to any one of the preceding claims, **characterized by** the fact that the composite structure includes a layer (115) that is impermeable or partially impermeable.

28. A device according to claim 27, **characterized by** the fact that the impermeable or partially impermeable layer is present between the matrix and the outside of the cover.

29. A device according to any one of the preceding claims, **characterized by** the fact that the composite structure has at least two juxtaposed or superposed adhesive matrices (IIa, IIb, IIc, IId, IIe, IIf) of compositions that are identical or different.

30. A device according to any one of claims 1 to 18, **characterized by** the fact that the composite structure comprises, in order, the fowling superposition of layers: a first solvent-permeable layer (I), a first adhesive matrix (IIa), a second adhesive matrix (IIb), and a second solvent-permeable layer (III).

31. A device according to any one of claims 1 to 18, **characterized by** the fact that the composite structure comprises, in order, the following superposition of layers: a first permeable layer (I), a first adhesive matrix (IIb) containing at least one active agent, an impermeable intermediate layer (115), a second adhesive matrix (IIa) containing at least one active agent, and a second permeable layer (III), the first and second adhesive matrices containing different active agents.

32. A device according to any one of the preceding claims, **characterized by** the fact that the sheet (11, 12) is arranged to enable the active agent to diffuse preferentially towards one side of the cover.

33. A device according to claim 32, **characterized by** the fact that the sheet is arranged to enable the active agent to diffuse preferentially towards the inside of the cover.

34. A method of manufacturing a device for treating a part of the body according to any one of the preceding claims, the method comprising the following steps:
· providing at least one sheet (11, 12; 21, 22; 21'; 31; 41, 42; 61, 62) comprising a composite structure of at least one adhesive matrix (II) present between two layers (I, III), at least one of which is permeable to a solvent, these two layers being bonded in permanent manner to the adhesive matrix, the adhesive matrix containing at least one active agent soluble in the solvent, and suitable once dissolved for being released through at least one side of the sheet; and
· folding said sheet over onto itself or shaping it into a bag or assembling it with at least one other sheet so as to create a cover presenting a cavity into which a part of the body can be inserted.

35. A method of cosmetically treating a part of the body, the method comprising the following step:
· introducing the part of the body to be treated into the cavity of the cover of a treatment device made in accordance with any one of claims 1 to 33, except claim 17, before or after introducing the solvent.

36. A method according to claim 35, **characterized by** the fact that massaging movement is imparted between the cover and the part of the body inserted inside it.

37. A method according to claim 35 or 36, **characterized by** the fact that, after a first treatment, the cover is turned inside-out and treatment is started over using the inside-out cover.

38. A glove for treating the hands, the glove being constituted by the cover of a device as defined in any one of claims 1 and 2 and 4 to 33.

39. A glove according to the preceding claims, **characterized by** the fact that it comprises at least one moisturizing agent.

40. A glove according to claim 39, **characterized by** the fact that it comprises at least one anti-chapping, antiherpes, burn-care, or eczema-care agent, or an agent that promotes healing.

41. A method of cosmetically treating the hands, the method comprising the following step:
· inserting the hand into a glove as defined in any one of claims 38 or 39 without dependency of claim 17, before or after introducing the solvent.
